# EUROPEAN PATENT APPLICATION

(11) **EP 1 619 242 A1**
(43) Date of publication of application: **25.01.2006**
(21) Application number: 04722990.1
(22) Date of filing: 24.03.2004
(51) Int. Cl.: C12N 5/06, A61K 35/28, A61P 43/00, G01N 33/15

(54) **CONTROL OF STEM CELL DIFFERENTIATION INDUCTION AND DIFFERENTIATION POTENCY**

(30) Priority: 25.03.2003 JP 2003083106; 31.03.2003 JP 2003095242
(71) Applicant: Japan Science and Technology Agency, Kawaguchi-shi, Saitama 332-0012 (JP)
(72) Inventor: OBINATA, Masuo, Miyagi 980-0871 (JP); SUZUKI, Yoshihisa, Miyagi 981-0905 (JP)
(74) Representative: Hiebl, Inge Elisabeth
(86) International application number: PCT/JP2004/004090
(87) International publication number: WO 2004/085632

(57) **Abstract**

This invention provides a method to induce the differentiation of animal cells wherein said cells are brought into contact with cytokine only in a suitable growth phase of animal cells, and also provides a system to control the differentiation potency of animal cells wherein two or more cytokines are combined for use in the cultivation or growing of animal cells.

## Description

### Technical Field

This invention relates to the cultivation, maintenance and proliferation, and also the induction of differentiation as well, of multipotent stem cells. In more detail, this invention relates to a method which is applicable to the technical field of regenerative medicine.

### Background Art

Traffic accident, injuries or diseases may cause a loss of tissue or organ such as heart, lung, kidney, blood vessel, alimentary tract, nerve, or the like. Thus, there have been demands for the development of technique of regenerative medicine by which to restore the lost tissue such as heart and blood vessel, utilizing not organ of other person but cells of the person who lost the tissue or organ, in detail, stem cells which keep function to differentiate into the lost tissue such as heart and blood vessel, or functional cells.

Under these circumstances, with regard to heart for example, there have been isolated a lot of important genes which take part in the development and differentiation of heart. There has also been conducted analysis of the development and differentiation of cardiomyocytes with use of pluripotent cells such as embryonic stem cells (ES cells). Furthermore, it has been suggested that stem cells including myeloid cells have multipotency to differentiate into various kinds of cells. It has also been reported that such bone marrow-derived cells contributed to the regeneration of damaged cardiac muscles in a living mouse and thus improved cardiac function.

In more detail, with respect to the analysis of the development and differentiation of ES cells, it is known that embryoid bodies generated from undifferentiated murine ES cells which have been primed with transforming growth factor β1 (member of TGF-β superfamily, i.e., TGF-β and BMP2), a kind of cytokine, demonstrate an increased potential for cardiac differentiation with a significant increase in beating areas (see, for instance, Non-Patent Document 1 below).

There have been examined actions of cytokine, as an important regulatory factor for growth and differentiation as stated above, on various kinds of cells. As a result, it is known that a cytokine has diverse functions, and that said functions are redundant.

With regard to bone marrow-derived cells, on the other hand, it has been reported that bone marrow stromal cell lines (TBR cell lines) established from temperature-sensitive SV-40 T-antigen gene transgenic mice exhibited myogenic, osteogenic and adipogenic differentiation. One of those stromal cell lines differentiates into skeletal muscles, and its differentiation is stimulated by oncostatin M (OSM), whereas the differentiation of another TBR cell line into smooth muscles is inhibited by OSM (see Non-Patent Document 2 below). Incidentally, general processes for the production of TBR cell lines, and details of the cell lines as well, have been published (see, for instance, Patent Document 1 and Non-Patent Documents 3 and 4 below). It is mentioned in Non-Patent Documents 3 and 4 that TBR cell lines exhibit phenotypic changes depending on the inactivation of T-antigen and growth condition; some preadipocyte line is induced toward adipocytes and osteoblasts, and some preadipocyte and endothelial cell lines are induced toward muscle cells and adipocytes. These results indicate that TBR cells are derived from multipotent mesenchymal stem cells. It is also known that mesenchymal stem cells can be induced toward osteocytes, chondrocytes, tendon cells, ligament cells, skeletal muscle cells, adipocytes, stromal cells, etc. (see Non-Patent Document 5 below).

Furthermore, it has been published that bone marrow-derived cells which have a potency of differentiation into cardiomyocytes are stochastically differentiated into each cell line of cardiomyocyte, adipocyte and skeletal muscle cell by the administration of DNA-demethylating agent such as 5-azacytidine (see Patent Document 2 below).

It is mentioned in Patent Document 2 that the addition of a combination of a cytokine among the four of FGF-8, ET1, Midkine and bone morphogenetic protein-4 (BMP4) and 5-azacytidine prompts bone marrow-derived cells to express cardiac muscle-specific gene. Patent Document 2 also suggests that, when murine myeloid cells which have a potency of differentiation into cardiomyocytes are previously treated with 5-azacytidine and are then transplanted into a mouse, cells derived from the transplanted cells are seen in cardiomyocytes and blood vessel cells.
- Patent Document 1:: Japanese Patent KOKAI Publication No. Hei 5-292958
- Patent Document 2:: Pamphlet of 01/48151, in particular page 4, lines 2-11; and pages 53-55
- Non-Patent Document 1:: The FASEB J. 2002; 16: 1558-1566, Abstract
- Non-Patent Document 2:: In Vitro Cell. Dev. Biol. - Animal 37: 698-704 (2001)
- Non-Patent Document 3:: J. Cellular Physiology 164: 55-64 (1995)
- Non-Patent Document 4:: Experimental Cell Research 218, 424-429 (1995)
- Non-Patent Document 5:: Science 284, 143-147 (1999)

As stated above, it is known in this art that not only ES cells but also certain kinds of bone marrow-derived cells (except hematopoietic stem cells) have pluripotency, and express a specific differentiated trait to become functional cells, and further to be tissues and organs. It is also suggested that specific trait and/or function and the direction of differentiation may change. Incidentally, it is mentioned also in Patent Document 2 that the direction of differentiation (expressivity of new differentiated trait) may be varied by the use of cytokine. The method as mentioned in Patent Document 2, however, essentially needs the combined use of 5-azacytidine (which is phosphorylated in a living body and taken into nucleic acid, and thus inhibits the synthesis of DNA) in principle. Besides, if there were provided a means to regulate the direction of differentiation more accurately, it would contribute to the progress of this art.

### Disclosure of Invention

The inventors of this invention have assiduously studied about the influence of cytokine on the direction and degree of differentiation of multipotent stem cells, and have found out that the above-mentioned diversity of function of cytokine is observed not only between different cells but also between different specific growth phases of cells of the same cell line. They have also found out that the direction of differentiation of animal cells can be regulated by use of a combination of two or more kinds of cytokine. This invention has been accomplished on the basis of these findings.

This invention provides a method to induce the differentiation of multipotent stem cells by bringing said cells into contact with a pharmacological agent during the process of growth of said multipotent stem cells, wherein said cells are brought into contact with the agent in at most four of the growth phases which comprise i) 1^{st} development stage, ii) 2^{nd} development stage, iii) the first period of 3^{rd} development stage, iv) the latter period of 3^{rd} development stage, v) the first period of 4^{th} development stage, and vi) the latter period of 4^{th} development stage, and wherein said agent is a substance which is capable of promoting and/or inhibiting the differentiation of said cells in at least two directions.

As another embodiment, this invention provides a method to evaluate the ability of a proposed agent to promote and/or inhibit the differentiation of cells with use of the above-mentioned method to induce the differentiation of cells wherein said proposed agent (or analyte) is brought into contact with said cells in at most four of the above-mentioned growth phases of multipotent stem cells. Such an evaluation method is usable, not restrictively, for the screening of substances (of peptide or non-peptide property) having an action similar to, or superior to, that of cytokine which, as mentioned later, is capable of regulating the proliferation and/or differentiation of multipotent stem cells.

This invention further provides a set of cytokines to regulate the differentiation of cells of mammals, which comprises a combination of two or more cytokines as an effective ingredient, and which is capable of determining three or more directions of differentiation of multipotent stem cells, e.g., bone marrow stromal cells, and is capable of regulating the degree of differentiation in each cells whose differentiation direction has been determined.

Such a set of cytokines as mentioned above is usable under circumstances which are selected from the group consisting of *in vitro*, *ex vivo* and *in vivo*. Embodiments of use as stated above include a method to screen cytokine which is to be employed as the above-mentioned ingredient, and other agents which act similarly to cytokine, and direct or indirect regenerative medicine, e.g., a method wherein, when multipotent cells are to be transplanted into a living body, the above-mentioned set of cytokine is administered in combination with said cells; a method wherein cells which have been taken from recipient *per se* are previously treated with the above-mentioned set of cytokine so that desired differentiated trait may be expressed; and a method wherein cells are transplanted into a living body after they have been differentiated into cells or tissues which exhibit specific function.

As a preferable embodiment of the above-mentioned screening, this invention provides a method for the screening of medicines which act on the differentiation potency of vertebrate cells, wherein (A) multipotent stem cells, in particular multipotent bone marrow stromal cells derived from temperature-sensitive SV-40 T-antigen gene transgenic mice are prepared, (B) said cells are cultivated in a medium in which the cells may be proliferated in the presence of an agent which is expected to be able to differentiate the cells, (C) the direction or degree of differentiation of thus cultivated cells is determined, and (D) the result of thus determined direction or degree of differentiation is compared with the result of cultivation of said cells in the absence of said agent, and, then, the difference between said two results is used as an index to the action of said agent on the differentiation potency of bone marrow stromal cells.

A more preferable embodiment includes a stage where cytokine selected from the group consisting of the above-mentioned BMP-2, BMP-4, OSM, GDF-5 and TGF-β2 is used as a comparative agent.

### Brief Description of Drawings

Figure 1 (a) is a photograph of cells in place of a drawing, which shows the result of induction of differentiation of TBR32-2; Figure 1 (b) is a photograph of cells in place of a drawing, which shows the result of induction of differentiation of TBR10-1.
Figure 2 is a graph which shows growth curve and development stage of TBR32-2.
Figure 3 is a graph which shows growth curve and development stage of TBR10-1.
Figure 4 (a) is a photograph in place of a drawing, which shows the result of western blotting after gel electrophoresis with regard to Examples 1-5 and Comparative Example 1, and Figure 4 (b) is a photograph in place of a drawing, which shows the result of western blotting after gel electrophoresis with regard to Examples 6-12.
Figure 5 (a) is a photograph in place of a drawing, which shows the result of western blotting after gel electrophoresis with regard to Examples 13-17 and Comparative Example 2, and Figure 5 (b) is a photograph in place of a drawing, which shows the result of western blotting after gel electrophoresis with regard to Examples 18-24 and Comparative Example 3.
Figure 6 is a photograph in place of a drawing, which shows the result of western blotting (protein was separated by SDS-polyacrylamide gel electrophoresis, and thus separated protein in the gel was transblotted onto membrane) with regard to Example 25. The upper photo: Smooth muscles; The lower photo: Skeletal muscle

### Best Mode for Carrying Out the Invention

Multipotent stem cells which are usable in this invention include ES cells and any other cells such as those which have been isolated from bone marrow, brain, liver and other organs and also said cells' primary culture cells, subculture cells and immortalized cells as well, so long as native cells express at least two new differentiated traits to become functional cells, tissues or organs by the action of some agent or other. Preferably used stem cells are, however, bone marrow-derived mesenchymal cells. Isolated or established cell lines which are preliminarily called bone marrow stromal cell or mesenchymal stem cells are also included in what is called multipotent stem cells in this invention so long as they serve to achieve the objective of this invention.

Thus, the cells as recited in the above-mentioned Non-Patent Documents 3 and 4 which show multipotency are to be taken as concrete examples. Furthermore, cells whose special properties are equivalent, in the direction and degree of differentiation, to those of the cells as mentioned in Non-Patent Documents 3 and 4 are also usable regardless of their origin. Specific examples of such cells are cells derived from mammals like mouse, rat, guinea pig, rabbit, cat, dog, sheep, goat, horse, pig, ox, monkey and human being. Although not restrictive, what are called TBR cell lines established from temperature-sensitive SV-40 T-antigen gene transgenic mice as mentioned in the above Non-Patent Documents 3 and 4 can be taken as an example of convenient multipotent cells from the viewpoint of confirming the action and effect of this invention. Incidentally, Dr. Masuo Obinata, one of the inventors of this invention, is included in co-authors of said Non-Patent Documents 3 and 4. Thus, specific cell lines of the above-mentioned TBR cell lines such as TBR31-2 and TBR10-1 are distributed, without restriction, by Institute of Development, Aging and Cancer, Tohoku University, Seiryo-cho 4-1, Aoba-ku, Sendai-shi, on the premise that the cell lines are to be used for experiment or research.

Growth processes in multipotent stem cells as explained above may be performed in any of the following: *in vitro, ex vivo* and *in vivo*. Attention has been paid, however, on *in vitro* and *ex vivo* growth processes in particular. Such cell's growth processes include various stages of development (or growth phases) in artificial cultivation of cells, and concretely comprise the following stages:
i) 1^{st} development stage: There are observed young and unmatured cells which are extending cell processes in all directions, and which have not yet entered growth phase.
ii) 2^{nd} development stage: There are observed cells which are in a stage immediately before the increase of the number of cells, and are extending cell processes in all directions; the cells are in a stage immediately before or after cell processes of adjacent cells begin to come into contact with one another; and the expansion of cell volume is at its maximum.
iii) the first period of 3^{rd} development stage: There are observed cells which begin to grow logarithmically; in other words, from a state where cell processes of at least 90 % of adjacent cells have already come into contact with one another, up to a state of 60 % confluent, i.e., cell density is 60 when a state is supposed to be 100 where main body of cells are perfectly in contact with one another and there is no room for further increase.
iv) the latter period of 3^{rd} development stage: There are observed cells from a state of 60 % confluent up to 100, which are going toward maturity.
v) the first period of 4^{th} development stage: There are observed cells in a state of 100% confluent, which are still slightly growing and immediately before the stop of growing.
vi) the latter period of 4^{th} development stage: There are observed matured cells which have finished the growth stage.

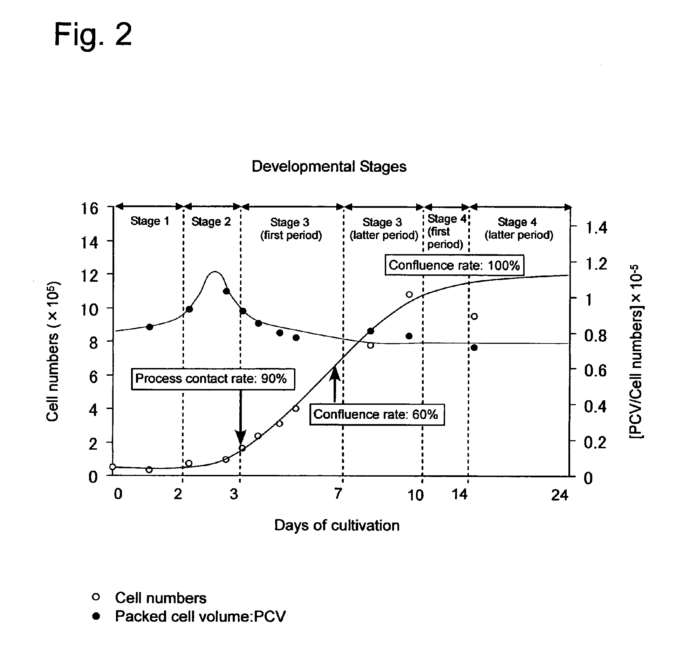

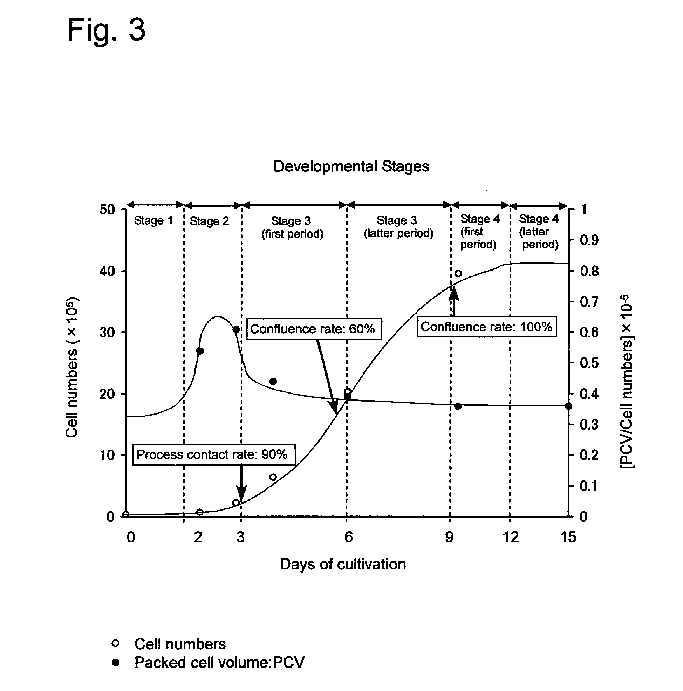

See also Figures 2 and 3.

In conventional techniques where cytokine is used, cells are cultivated, through the whole period of cultivation, in a cytokine-containing medium, and, therefore, cells are continuously kept in contact with (or exposed to) cytokine from the initial period of cultivation up to the end of cultivation. In this invention, on the other hand, it is important that cells should be kept in contact with a substance (preferably one of cytokines), which is capable of promoting and/or inhibiting the differentiation of cells in at least two directions, in at most four of the above-mentioned growth phases i) to vi). Culture medium is renewed, for instance every two to four days in a process to grow cells, typically during the cultivation of cells. Whether or not to achieve such contact as mentioned above can easily be controlled by whether allowing the above-mentioned substance to be present or not in the new medium. Said at most four growth phases comprise not only, for instance, iii) the first period of 3^{rd} development stage, iv) the latter period of 3^{rd} development stage, v) the first period of 4^{th} development stage, and vi) the latter period of 4^{th} development stage, but also three phases of, for instance, i) 1^{st} development stage, ii) 2^{nd} development stage, and iii) the first period of 3^{rd} development stage, or also two phases of, for instance, i) 1^{st} development stage and ii) 2^{nd} development stage, or iv) the latter period of 3^{rd} development stage and v) the first period of 4^{th} development stage, and, furthermore, a single phase among the above-mentioned six.

According to this invention, preferably one species of cytokine is added, in such a concentration that the induction of differentiation may be caused to cells which are being grown, to a medium in one of the above-mentioned combination of phases. Examples of such cytokine include, although not restrictive, oncostatin M (OSM), bone morphogenetic protein-2 (BMP-2), bone morphogenetic protein-4 (BMP-4), growth differentiation factor 5 (GDF-5) and transforming growth factor (TGF-β2).

According to Non-Patent Document 2 for instance, when the above-mentioned TBR31-2 is continuously kept contact with OSM through the whole culture period (i.e., the above-mentioned phases i) to vi)), osteogenic differentiation is stimulated (or the induction of differentiation toward osteoblasts is promoted) as stated above. Adipogenic differentiation is, however, inhibited (or the induction of differentiation toward adipocyte is inhibited). According to this invention, on the other hand, the induction of differentiation from TBR31-2 toward adipocyte is inhibited not only when OSM is made present in the above-mentioned phases iv) to vi), but also in a single phase of vi). A more important finding is that, as mentioned in Non-Patent Document 2, when TBR31-2 is cultivated in the presence of OSM through the above-mentioned growth phases i) to vi), the induction of differentiation toward smooth muscle cells is inhibited, whereas, when TBR31-2 is cultivated in the presence of OSM in the growth phases i) to iii), or even in a single phase of iii), iv) or v), the induction of differentiation toward smooth muscles cell is promoted.

Thus, when the action of cytokine on multipotent stem cells is to be evaluated in a growth system of multipotent stem cells, the direction of induction of differentiation may become opposite depending on the timing of contact between said cells and cytokine. Therefore, when the action of agents such as cytokine on multipotent stem cells is to be accurately confirmed, it is preferable that confirmation should be carried out in any suitable one to four growth phases among the above-mentioned six growth phases i) to vi).

Hence, when the method of this invention to induce the differentiation of multipotent stem cells is applied to the evaluation of the action of various proposed agents (or analytes) on the cells, it becomes possible to precisely evaluate the agents for their ability of induction of differentiation. For such an evaluation method, it is convenient to use bone marrow stromal cells (TBR cell lines) established from temperature-sensitive SV-40 T-antigen gene transgenic mice, since said cells are easy to cultivate and also easy to evaluate the result of cultivation. A preferable, or more detailed embodiment, of such an evaluation method comprising:
A) when TBR cell lines are to be grown in their specific nutritious medium, TBR cells are cultivated in at most four of the growth phases in said cells' growth process or cultivation which are composed of i) 1^{st} development stage, ii) 2^{nd} development stage, iii) the first period of 3^{rd} development stage, iv) the latter period of 3^{rd} development stage, v) the first period of 4^{th} development stage, and vi) the latter period of 4^{th} development stage, in the presence of analyte in a suitable concentration;
B) it is detected whether or not thus cultivated cells have transformed into cells which have any differentiated trait (or whether or not differentiation induction has occurred in the cultivated cells);
C) it is judged, from the result of detection, whether or not the analyte has an ability of induction of differentiation of multipotent stem cells, or has an action of promoting or inhibiting the differentiation of said cells.

In such an evaluation method as mentioned above, the action of analyte on multipotent stem cells can be more detailedly evaluated by including a step, as a comparison or positive control, where a comparison is made with the result of cultivation in the absence of said analyte and in the presence of a cytokine selected from the group consisting of bone morphogenetic protein-2 (BMP-2), bone morphogenetic protein-4 (BMP-4), oncostatin M (OSM), growth differentiation factor 5 (GDF-5) and transforming growth factor (TGF-β2).

In this invention which uses a combination of two or more cytokines, on the other hand, cytokines may be used either simultaneously or separately with regard to time, site, etc. In another case, cytokines have once been used in combination (or as a set), and, afterward, only one of the cytokines may be used; such a case is also included in the use of a set of cytokines of this invention. For instance, there may be a case where two or more cytokines are used for the purpose of determining the direction of differentiation of multipotent stem cells and controlling the degree of the direction, and, thereafter, a single cytokine is used for selective growth and differentiation with a view to obtaining differentiated specific cells in accordance with the ultimate objective. In this case also, the whole of the stages is understood to come under the use of a set of cytokines of this invention. Differentiation in three or more directions means that, when multipotent stem cells are grown, three or more new characters (differentiated traits) are expressed or three or more functional cells, tissues or organs are directed; plainly speaking, a property is meant where there are directed some functional cells or other, e.g., smooth muscle cells or skeletal muscle cells. Thus, the degree of differentiation means the size of appearance rate of, for instance, smooth muscle cells or skeletal muscle cells in comparison with a control, e.g., appearance rate of each of differentiated cells with no addition of isolated cytokine and in the presence of serum.

Direction of differentiation as mentioned above means a property that multipotent stem cells express a differentiated trait of, or are directed to, smooth muscle cells, skeletal muscle cells, cardiomyocytes, endothelial cells or adipocytes, which are however not restrictive. When a set of two or more cytokines of this invention is used, multipotent stem cells can be directed to at least three of the above-mentioned differentiated cells, and, moreover, the degree of expression of differentiated trait or the degree of appearance of thus obtained differentiated cells can be controlled. Said degree means detectable promotion (increase) or inhibition (decrease) in comparison with the degree of expression of differentiated trait or the degree of appearance of the obtained differentiated cells where multipotent stem cells are cultivated in substantially the same medium with regard to culture composition except that, whereas cytokine is not added, serum is supplemented instead. Said degree is preferably promotion or inhibition by at least 10 % in comparison with the above-mentioned degree of expression of differentiated trait or the degree of appearance of the obtained differentiated cells in the presence of serum. Thus, this invention can selectively raise the rate of appearance or the proportion of existence of any specific differentiated cells. As to how to detect the above-mentioned degree of expression of differentiated trait or the degree of appearance of differentiated cells, some specific methods therefor are given in Examples which are mentioned later. However, any method or technique known in this art is usable.

In this invention, any cytokine is usable to constitute the combination (or set) of at least two cytokines which can induce multipotent stem cells in a manner as stated above, and, moreover, cytokines may be combined in any way, so long as the cytokine serves to achieve the objective of this invention, i.e., to selectively enhance the appearance rate or the proportion of existence of some specific differentiated cells. Examples of such a cytokine, although not restrictive, include bone morphogenetic protein-2 (BMP-2), bone morphogenetic protein-4 (BMP-4), oncostatin M (OSM), growth differentiation factor 5 (GDF-5) and transforming growth factor (TGF-β2). Examples of combination of these cytokines include BMP-2 and BMP-4; BMP-2 and OSM; BMP-2 and TGF-β2; BMP-2, BMP-4 and OSM; OSM-BMP-4; OSM and TGF-β2; OSM and GDF-5; OSM, GDF-5 and BMP-4; OSM, GDF-5, TGF-β2 and BMP-4; BMP-2, OSM, GDF-5 and BMP-4; and BMP-2, OSM, GDF-5, TGF-β2 and BMP-4. For instance, it is confirmed that a combined use of BMP-2 and OSM is capable of differentiating certain multipotent stem cells (see Examples which are mentioned later) into autonomically beating cardiomyocytes or into endothelial cells, each at a high appearance rate. Thus, BMP-2, OSM or both are to be applied to stem cells having such pluripotency, depending on the purpose of differentiation.

In the above-mentioned method of induction of differentiation or method of evaluation, or in a system of combined use of at least two cytokines, any known condition for cultivation of animal cells such as TBR cell lines can be employed as it is or with a modification. For instance, any medium is basically usable, so long as it contains essential nutrient source; in detail, as for amino acid, so long as it contains, in its base, essential amino acid which animals (mammals) cannot synthesize such as L-arginine, L-cysteine, L-tyrosine, L-histidine, L-isoleucine, L-leucine, L-lysine, L-methionine, L-phenylalanine, L-threonine, L-tryptophan, L-valine and L-glutamine, and, if necessary for better growth, also non-essential amino acid such as L-glycine and L-serine as well. As a buffer to add for the purpose of stabilizing pH of medium in cooperation with gaseous CO₂ in CO₂ incubator, there may be added a solution of sodium bicarbonate or HEPES as well since cells need hydrogencarbonate ion, so that the medium may keep its pH stable even though taken out of CO₂ incubator. Furthermore, antibiotics may be added where necessary, with a view to preventing contamination by unwanted bacterium. In this invention, serum and other pharmacological agents are removed from medium where necessary, and cytokine is added instead. *In vitro*, on the other hand, there can be employed an embodiment wherein cytokine is included in a medium which has no adverse effects either physiologically or on cytokine used. Examples of said medium, although not restrictive, include sterile water, physiological saline and phosphate-buffered saline.

As for the amount of cytokine which is to be used *in vitro* or *ex vivo,* optimal amount may be determined in consideration of the amount which is mentioned in Examples as given later, or, if necessary, further by conducting small scale experiments. When used *in vivo*, the amount of cytokine is usually 0.1 ng/ml to 20 ng/ml. Cultivating temperature may be 33°C to 37°C.

Cells which have been induced to differentiation can be identified by any known method. Specifically, methods which are mentioned in Examples as given later can be followed.

As another embodiment, this invention provides a preparation for regenerative medicine which mainly comprises differentiated cells that have been obtained by the aforementioned methods of induction of differentiation. Thus, this invention provides means which are applicable to medicine to regenerate vascular organs such as heart, blood capillary, artery and vein; digestive organs such as stomach, esophagus, colon, rectum, small intestine and large intestine; muscle of extremities, trachea, uterus, vagina and adipose tissue.

Moreover, the above-mentioned evaluation method can construct a system for screening pharmacological agents which can control the degree of differentiation.

A set of at least two cytokines, on the other hand, may be made to exist, either in combination or not with the above-mentioned sterile water, physiological saline and phosphate-buffered saline, in a matrix of material which has no adverse effects on a living body, to be used for controlled release. Examples of material which constitutes said matrix include polylactic acid (porous material), collagen, collagen sponge, β-calcium triphosphate, porous hydroxyapatite, polylactic acid microsphere, polylactate-coated gelatin sponge, lactic acid-ethyleneglycol copolymer, agarose polyvinyl alcohol, alginic acid, agarose/heparin, amylopectin, fibringel, collagen minipellet, ethylene-vinyl acetate copolymer, lactic acid-glycolic acid copolymer, chitosan, lactic acid/glycolic acid-ethyleneglycol copolymer and titanium implant.

Besides, when at least two cytokines are combined or used as a set in vitro, there can be constructed a system for the screening of pharmacological agent which can determine the direction of differentiation of multipotent cells, and which can furthermore control the degree of each differentiation whose direction has been determined, with use of the above-mentioned TBR cell lines which can be used and manipulated easily, and which have been provided by a part of inventors of this invention. In such a screening system, a set of cytokines of this invention is usable as a comparative specimen. For instance, multipotent stem cells are cultivated in the presence of one or more of the above-mentioned cytokine, instead of carrying out cultivation in the presence of proposed substance for pharmacological agent to be screened (e.g., proposed agent which is expected to induce TBR cell lines to differentiate), and, thus, the result of cultivation is used as a standard (or criterion) for evaluating the action of said proposed substance. In this way, any pharmacological agent that may serve to induce the differentiataion of multipotent stem cells in a predetermined direction can efficiently be screened. Incidentally, the above-mentioned cultivation conditions can be applied to such a screening system.

In the following, this invention is explained in more detail, but not restrictively.

(1) Identification or detection of cells which are induced to differentiation or have been differentiated:
(1-1) With regard to smooth muscle cells, mononuclear and spindle-shaped cells were microscopically observed in a population of cells which had been cultivated in a 60 mm-plastic dish. For the purpose of detecting, from among a protein mixture which had been extracted from the population of cultivated cells, myosin L chain kinase (sm-MLCK) or α-actin (α-sm-actin) which specifically expresses in smooth muscle cells, protein was separated by SDS-polyacrylamide gel electrophoresis, and thus separated protein in gel was transblotted onto membrane (WB method), and, thus, smooth muscle cells were identified with use of either primary antibody (monoclonal anti-myosin light chain kinase manufactured by Sigma, mouse IgG 2b, clone K36, Product No. M7905) which specifically reacted with sm-MLCK on the membrane, or primary antibody (monoclonal anti-α Smooth muscles Actin manufactured by Sigma, mouse IgG 2a isotype, clone 1A4, Product No. A2547) which specifically reacted with α-sm-actin, and with use of horseradish peroxidase (HRP)-labeled secondary antibody (goat, anti-mouse IgG F(ab')2, manufactured by ICN, IgG Catalogue No. 55553) by which to detect reacted primary antibody.
(1-2) For the purpose of observation of cell nuclei in cultivating dish, propidium iodide (PI) as a staining solution whose concentration had been regulated to 1 µg/ml was added after culture medium had been removed, and, after the treatment with staining solution for five minutes at a room temperature, the staining solution was removed, and, then, cells were washed with phosphate buffer solution (PBS). Rinse with PBS was conducted three times, each for five minutes.
(1-3) As for ossified cells, it is known that alkaline phosphatase (hereinafter referred to as ALP) exists in osteoblasts in a considerably high concentration, and that calcium in the form of hydroxyapatite is deposited in osteoblasts. Hence, ossified cells were identified by the former ALP staining method and by the latter von Kossa's method. The detection of this HRP-labeled secondary antibody was conducted by the exposure of X-ray film (XAR-5 manufactured by Kodak) to chemiluminescence made by chemiluminescence-detecting reagent (ECL Plus Western Blotting Detection Kit manufactured by Amersham Bioscience K.K.). Firstly, with respect to ALP method, culture medium was removed from a 60 mm-plastic cultivating dish, and, then, cells adhered to dish bottom were washed with PBS three times, each for three minutes. Thereafter, the cells were left to stand still on ice for 10 minutes, and were thus fixed with 70 % cool ethanol solution. Then, after the cells were washed with distilled water three times, ALP reaction liquid as shown in Table 1 was poured, and, then, the cells were left to stand still at 37°C for four hours.

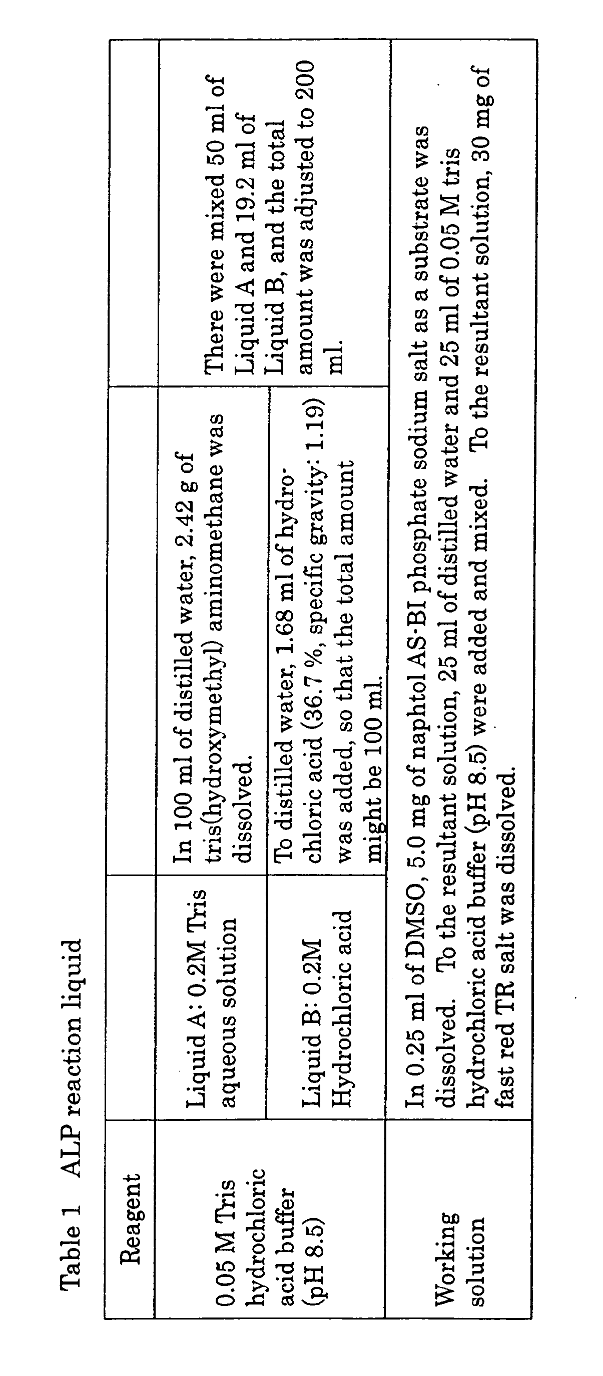

In the meantime, it was microscopically observed where necessary whether cells had been stained in pink or not. After the cells were washed with distilled water three times, Meyer's hematoxylin solution (solution for use of pathological research No. 131-09665 manufactured by Wako Pure Chemical Industries, Ltd.) was poured, and, then, the cells were left to stand still at room temperature for five minutes so that nucleus might be stained. After lightly washed with water, the cells were soaked in warm water for the purpose of coloration. Finally, the cells were washed with distilled water three times. With regard to von Kossa's method, culture medium was removed from a 60 mm-plastic cultivating dish, and, then, cells adhered to dish bottom were washed with PBS three times, each for three minutes. Thereafter, the cells were left to stand still on ice for 10 minutes, and were thus fixed with 10 % cool formalin solution. Then, the cells were washed with distilled water twice. There was poured 5 % silver nitrate solution, and a reaction was allowed to proceed until calcium deposited part became blackish brown under indirect light in a room. Then, the cells were washed with distilled water three times. Thereafter, 5 % sodium thiosulfate solution was poured, and the cells were left to stand still for three minutes for fixation. Then, the cells were washed with distilled water three times. Thereafter, Kernechtrot solution as shown in Table 2 was poured for the sake of counter staining. After left to stand still for five minutes, the cells were washed with distilled water three times. Evaluation of ossified cells was conducted by microscopic observation.

**Table 2 Von Kossa's method**

| Reagent | Process for the preparation |
|---|---|
| 5 % Silver nitrate solution (prepared immediately before use) | In 100 ml of distilled water, 5 g of silver nitrate was dissolved. |
| 5 % Sodium thiosulfate solution | In 100 ml of distilled water, 5 g of sodium thiosulfate was dissolved. |
| Kernechtrot solution | In 100 ml of boiling distilled water, 0.1 g of Kernechtrot (made by Merck) and 5 g of aluminum sulfate were dissolved, and, then, the resultant mixture was cooled and filtrated. |

(1-4) With regard to endothelial cells, cobblestone-shaped cells were microscopically observed in a population of cells which had been cultivated in a 35 mm-plastic dish. Endothelial cells were identified by the confirmation of their ability of the uptake of acetylated low-density lipoprotein labeled with 1,1'-dioctadecyl-3,3,3'-tetramethyl-indocarbocyanine perchlorate (DiI-Ac-LDL: Product No. L-3484, manufactured by Molecular Probes, USA). In more detail, culture medium in a cultivating dish was sucked up and disposed of, so that cells might not be injured, and, then, DiI-Ac-LDL which had been adjusted to 10 µg/ml was added to a serum-free medium, and, thus, the cells were cultivated therein at 33°C for four hours. DiI-Ac-LDL which had not been taken up by the cells was removed by washing with PBS three times, each for five minutes. Thereafter, a 3 % formaldehyde solution was added, and, subsequently, the cells were left to stand still at room temperature for 10 minutes, and were thereby fixed. Then, after washed with distilled water, the cells were observed by a fluorescence microscope with rhodamine filter.
(1-5) With regard to adipocytes, cells having lipid droplet were microscopically observed in a population of cultivated cells, and were identified as adipocytes by an oilred staining method. Culture medium in a cultivating dish was disposed of, and cells which had adhered to dish bottom were washed with PBS three times, each for three minutes. Thereafter, with a 3 % formalin solution, the cells were left to stand still at room temperature for 10 minutes, and were thereby fixed. Then, after the formalin solution was substituted by 80 % isopropanol, the cells were left to stand still for a period of one minute, and were subsequently stained with oil red O at 37°C for 10 minutes. After the oilred staining liquid was substituted with 60 % isopropanol, the cells were left to stand still for one minute, then washed twice, each for three minutes, and finally were treated with Meyer's hematoxylin solution (manufactured by Wako Pure Chemical Industries, Ltd.) for 10 minutes for the sake of nucleus staining. The cells were subsequently washed with running water for two minutes, and, then, adipocytes stained in red were microscopically identified. Incidentally, when grown and matured, adipocytes have remarkable lipid droplet. Hence, even without staining, it would have been easy to identify the cells as adipocytes.
(1-6) With regard to the measurement of cells, 1 ml of EDTA-trypsin solution (a solution of 0.02 g of EDTA·3Na/100 ml of PBS and 0.002 g of trypsin/100 ml of PBS) was added after culture medium had been removed, and the cells were thereby washed. This washing treatment was rapidly conducted twice before the cells were peeled off. In detail, trypsin solution was added, and, immediately after the trypsin solution was spread uniformly on the surface of the cells, the trypsin solution was sucked up quickly with a Pasteur pipette, and was thereby removed. Then, after it was microscopically confirmed that cells had been completely peeled off, 5 ml of RITC medium was added to 60 mm-plastic dish, and, subsequently, the peeled cells were washed by pipetting, and were then transferred to a 10 ml centrifuge tube, by which the number of cells per 60 mm-plastic dish was calculated. For this calculation, Elmer's Bürker-Türk hemocyto-meter was used.

As for the measurement of volume of cells (packed cell volume, or PCV), the number of cells per 4 ml of thus prepared solution was calculated. Then, 4 ml of the solution was put in a hematocrit centrifuge tube (each graduation represents 1 µl; total volume is 10 µl), and centrifuged at 1000 rpm for three minutes. From the indicated volume of precipitated cells, a volume per 10⁵ cells were calculated. The used centrifugal machine was CF7D manufactured by Hitachi Koki Co., Ltd.

### (2) Cultivation of cells:

### (2-1) Comparative cultivation

With use of 2 % FBS-containing RITC 80-7 medium as shown in Table 3 below (hereinafter referred to as RITC medium), cells were cultivated in a 90 mm-plastic dish up to sub-confluent state (or confluent of about 80 %), i.e., a state immediately before cells came into contact with one another. After culture medium was removed, 1 ml of EDTA·trypsin solution (a solution of 0.02 g of EDTA·3Na/100 ml of PBS and 0.002 g of trypsin/100 ml of PBS) was added, and the cells were thereby washed. This washing treatment was rapidly conducted twice before the cells were peeled off. In detail, trypsin solution was added, and, immediately after the trypsin solution was spread uniformly on the surface of the cells, the trypsin solution was sucked up quickly with a Pasteur pipette, and was thereby removed. Then, after it was microscopically confirmed that cells had been completely peeled off, RITC medium as shown in Table 3 was added again, and, thus, the concentration of cells was adjusted to 1.2 x 10⁴/ml. To 60 mm-plastic dish, 4.0 ml of the resultant cell suspension liquid was added. After uniformly dispersed, the cells were cultivated at 33°C.

**Table 3 RITC 80-7 medium**

| Item | Amount added per 1000 ml of Milli Q Ultrapure Water | Notes |
|---|---|---|
| Sodium hydroxide | 0.3 g | Manufactured by Wako Pure Chemical Industries, Ltd. (Extra Pure Grade) |
| Sodium bicarbonate | 1.4 g | Manufactured by Wako Pure Chemical Industries, Ltd. (Extra Pure Grade) |
| HEPES | 3.3 g | Sigma No. H-3375 |
| RITC 80-7 | 9.83 g | Iwaki Glass Co., Ltd. No. 99-591-PS |
| Transferrin (10 mg/ml) | 1 ml | Itoham Foods Inc. No. 30601293 |
| rh EGF (10 µg/ml) | 1 ml | RSD. No. 236-EG-200 |
| Insulin (1 mg/ml) | 1 ml | Shimizu Seiyaku Co., Ltd. Batch No. DC18B |
| FBS (fetal bovine serum) | 20 ml | Gibco. BRL. Lot No. A0247611 |

With regard to the effect of addition of cytokines on the differentiation potency of cells, RITC medium as shown in Table 3 above was used for cell cultivation, and, 12 hours after the start of cultivation, the medium was replaced with a new one. Medium was replaced every three or four days. Cytokines were added every time medium was replaced. The amount of cytokines added is shown in Table 4.

**Table 4 Amount of cytokines added**

| Kinds | Concentration as prepared | Amount added/4.0 ml of medium | Final concentration | Notes |
|---|---|---|---|---|
| BMP-2 | 10 µg/ml | 8 µl | (20 ng/ml) | Made by RSD |
| OSM | 25 µg/ml | 1.6 µl | (10 ng/ml) | Made by RSD |
| GDF-5 | 50 µg/ml | 0.8 µl | (10 ng/ml) | Made by RSD |
| TGF-β2 | 1 µg/ml | 20 µl | (5 ng/ml) | Made by RSD |
| BMP-4 | 10 µg/ml | 8 µl | (20 ng/ml) | Made by RSD |

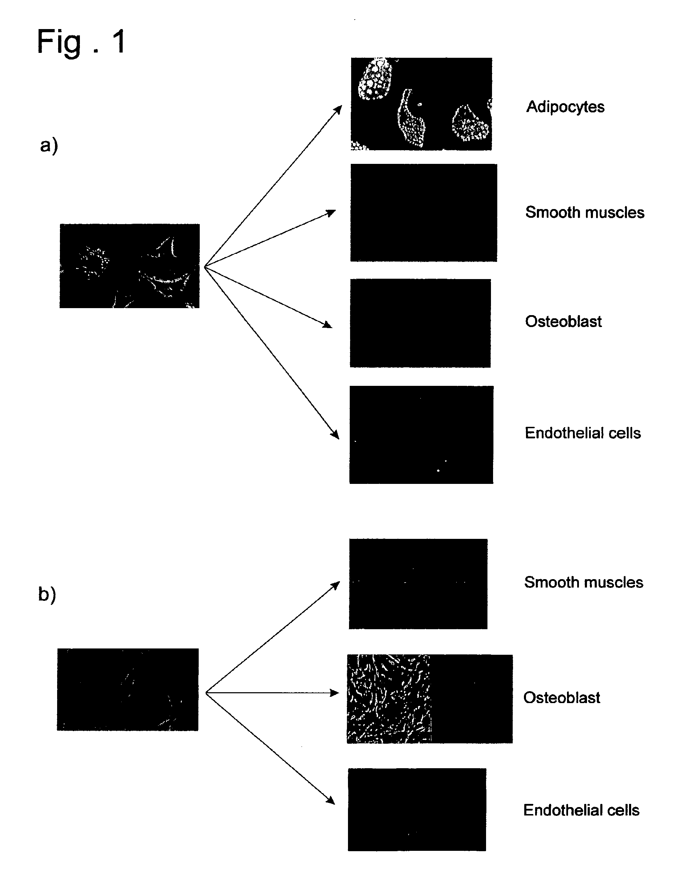

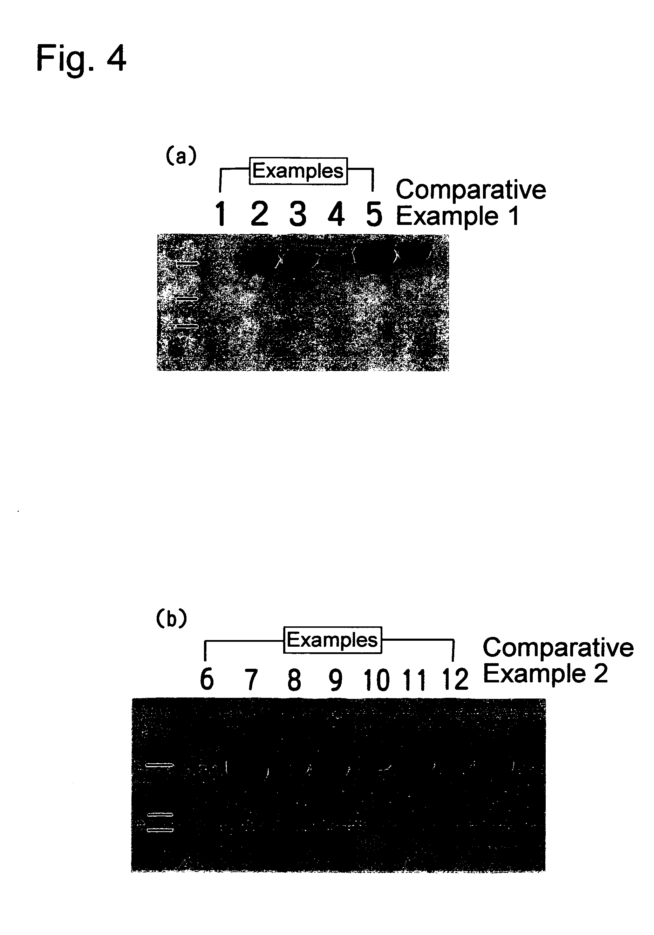

The differentiation of cells was judged by the following criterion. With regard to smooth muscle cells, mononuclear and spindle-shaped cells were microscopically observed, and, from among a protein mixture which had been extracted from the population of cultivated cells, the expression of myosin L chain kinase protein or α-actin protein which specifically expresses in smooth muscle cells was judged by the above-mentioned WB method. As for adipocytes, the number of cells having lipid droplet per unit area (3.8 mm²) was measured, and average was calculated.
(2-2) Cloned cell line of mesenchymal stem cells TBR31-2 which is used in Examples 1 to 12 (Examples 1 to 5 are referential examples) was established in 1995 as stated in the above-mentioned Non-Patent Documents 3 and 4, and was shown to have potency for differentiation toward adipocytes and osteocytes. The inventors of this invention have made a detailed research of cultivation conditions with use of said cell line together with α-MEM medium and high-concentration fetal bovine serum (10 % FBS) as mentioned in Table 5 below, and have clarified that said stem cells have potency for differentiation into four phenotypes including smooth muscles and endothelial cells as well as adipocytes and osteocytes (see Figure 1a and Table 6), and have further developed a technique with which the proportion of said differentiation can freely be changed with RITC medium which contains either no serum or a 0.7 % low-concentration FBS, and with cytokines (Examples 1 to 5). In detail, as shown by the results of expression of α-actin (see Table 6 and Figure 4a) as a marker of differentiation toward smooth muscle cells and by the results of differentiation toward adipocytes (Examples 1 to 5), OSM of Example 1 inhibits both differentiations toward smooth muscles and adipocytes, whereas BMP-2 of Example 2 and BMP-4 of Example 3 promote both differentiations toward smooth muscles and adipocytes. GDF-5 of Example 4, on the other hand, inhibits differentiation toward smooth muscles while promoting differentiation toward adipocytes. TGF-β2, on the contrary, promotes differentiation toward smooth muscles while inhibiting differentiation toward adipocytes.

**Table 5 α-MEM medium**

| Item | Amount added per 1000 ml of Milli Q Ultrapure Water | Notes |
|---|---|---|
| Sodium bicarbonate | 2.2 g | Manufactured by Wako Pure Chemical Industries, Ltd. (Extra Pure Grade) |
| α-MEM | 10.1 g | Manufactured by Wako Pure Chemical Industries, Ltd. (Extra Pure Grade) |
| 2-Mercaptoethanol (100 mM) | 0.25 ml | Sigma No. M-6250 |
| FBS (fetal bovine serum) | 1.00 ml | Gibco. BRL. Lot No. A0247611 |

**Table 6 Effects of cytokines on the differentiation of mesenchymal stem cells TBR31-2 toward smooth muscle cells, osteocytes and adipocytes (RITC 80-7, 37°C, 25 days)**

| Differentiation potency | Example 1 | Example 2 | Examp. 3 | Examp. 4 | Ex.5 | Compara. Ex.1 |
|---|---|---|---|---|---|---|
| | OSM (10 ng/ml) * | BMP-2 (20 ng/ml) | BMP-4 (20 ng/ml) | GDF-5 (100 ng/ml) | TGF-β2 (10 ng/ml) | |
| Smooth muscles (α-sm-actin) | - | + + + | + + + | ± | + + + + | + + |
| Adipocytes ** | 0 | 413 | 689 | 117 | 0 | 15 |

| | | | | | | |
|---|---|---|---|---|---|---|
| *: Parenthetical values show final concentration in medium to which each cytokine was added. | | | | | | |
| ** Average of the number of cells per 3.8 mm² which was measured at five places at random. | | | | | | |
| Examp. and Ex. denote Example, and Compara. Ex. denotes Comparative Example. | | | | | | |
| Marks: The degree of expression of α-sm-actin as a marker of differentiation toward smooth muscles as shown in Fig. 4(a), detected by chemiluminescence. + + + +: considerably strong expression; + + +: strong expression; + +: slightly strong expression; +: obvious expression; ±: feeble but obvious expression: -: no expression at all | | | | | | |

In this manner, the direction of differentiation can freely be controlled by combination of cytokines. In other words, undifferentiated cells can be stably maintained as they are, or can be amplified, and functionally differentiated cells can be maintained or amplified, with their function kept as it is.
(2-3) The above-mentioned control of differentiation was achieved by continuously giving cytokines. Cytokines, however, do not necessarily need to be continuously given. Control of differentiation can be achieved also by choosing timing of giving cytokines. This fact is explained further concretely in the following, with regard to TBR31-2 and TBR10-1 taken as examples.

### (2-3-1)

In the following, the development stage of TBR31-2 is explained in accordance with Figure 2.
1^{st} development stage: There are observed young and unmatured cells which are extending cell processes in all directions, and which have not entered growth phase.
2^{nd} development stage: There are observed cells which are in a stage immediately before the increase of the number of cells, and are extending cell processes in all directions; the cells are in a stage immediately before or after cell processes of adjacent cells begin to come into contact with one another; and the expansion of cell volume is at its maximum.
the first period of 3^{rd} development stage: There are observed cells which begin to grow logarithmically; in other words, from a state where cell processes of at least 90 % of adjacent cells have already come into contact with one another, up to a state of 60 % confluent, i.e., cell density is 60 when a state is supposed to be 100 where main body of cells are perfectly in contact with one another and there is no room for further increase.
the latter period of 3^{rd} development stage: There are observed cells from a state of 60 % confluent up to 100, which are going toward maturity.
the first period of 4^{th} development stage: There are observed cells in a state of 100% confluent, which are still slightly growing and immediately before the stop of growing.
the latter period of 4^{th} development stage: There are observed matured cells which have finished the growth stage.

Firstly, the effects of OSM on the differentiation of the above-mentioned cell strain toward smooth muscles and adipocytes are explained below. As mentioned above, Example 1 shows that OSM inhibits differentiation toward smooth muscles and adipocytes. The inhibition of differentiation toward smooth muscle cells and ossified cells is different from development stage to development stage. When OSM is given continuously for 24 days, differentiation toward smooth muscle cells is inhibited (Example 6, or a repetition of Example 1). This inhibition effect is also obtained by giving OSM only in and after the latter period of 3^{rd} development stage, in particular only in the latter period of 4^{th} development stage (Examples 8 and 12). When, however, OSM is given continuously from 1^{st} development stage to the first period of 3^{rd} development stage, differentiation toward smooth muscles is promoted on the contrary (Example 7). As for differentiation toward adipocytes, it is inhibited when OSM is given continuously for 24 days (Example 6). This inhibition effect is, however, seen in all the development stages except 3^{rd} development stage and the first period of 4^{th} development stage (Examples 7, 8, 9 and 12).

When, however, OSM is given from the latter period of 3^{rd} development stage to the first period of 4^{th} development stage, differentiation toward adipocytes is promoted (Examples 10 and 11).

In this manner, the inhibition of differentiation toward smooth muscle cells in the development stages of stem cells occurred in matured cells (4^{th} development stage) which had finished growth stage, and the inhibition of differentiation toward adipocytes occurred also in matured cells (the latter period of 4^{th} development stage) which had finished growth stage. The promotion of differentiation toward smooth muscle cells occurred in cells from unmatured state to logarithmic growth period when cells were actively proliferating (1^{st} to 3^{rd} development stages). The promotion of differentiation toward adipocytes occurred in cells going to maturity and immediately before the stop of growing (the latter period of 3^{rd} development stage to the latter period of 4^{th} development stage). See Table 7 (or Figure 4b) for the results.

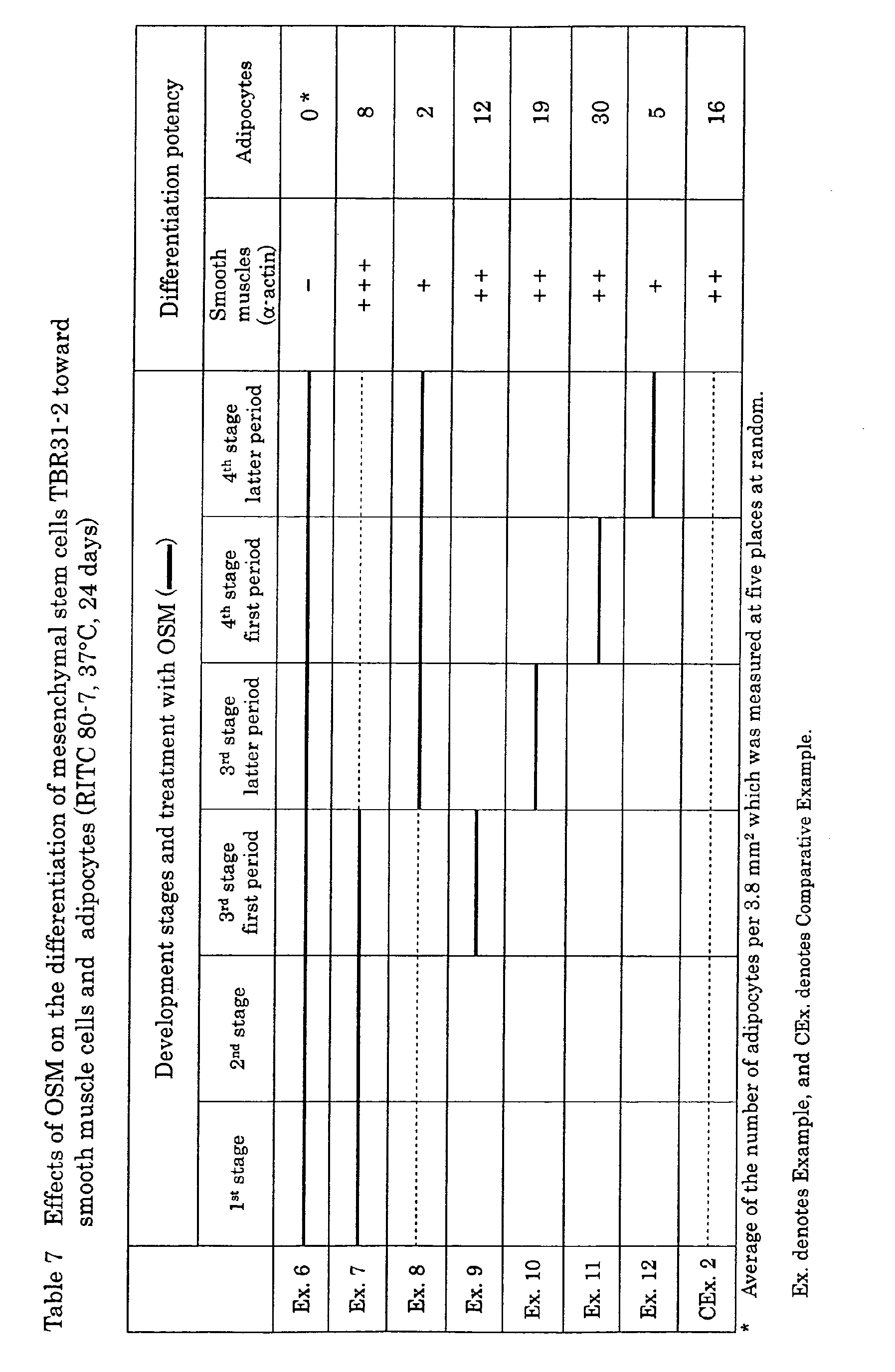

- Marks:: The degree of expression of α-sm-actin as a marker of differentiation toward smooth muscles as shown in Fig. 4(b), detected by chemiluminescence. + + +: strong expression; + +: slightly strong expression; +: obvious expression; -: no expression at all

As is seen in the above results, the effects of cytokines are different from stage to stage of development process of stem cells. Proportion of differentiation toward smooth muscle cells and adipocytes can be freely controlled by choosing timing of giving cytokines.
(2-3-2) The following is an explanation with regard to another cell line TBR10-1, taken as an example. Cloned cell line of mesenchymal stem cells TBR10-1 which is used in Examples 13 to 24 (Examples 13 to 17 are referential examples) and in Comparative Example 2 is as stated in the above-mentioned Non-Patent Documents 3 and 4, and was shown to have potency for differentiation toward smooth muscle cells (FEBS Letters 481: 193-196, 2000). The inventors of this invention have made a detailed research of cultivation conditions with use of said cell line together with α-MEM medium and high-concentration fetal bovine serum (10 % FBS) as mentioned in Table 5, and have found out that the stem cells differentiate toward ossified cells and endothelial cells as well as smooth muscle cells, and so have clarified that said stem cells are multipotent stem cells having potency for differentiation into three phenotypes (see Figure 1b and Table 8). Furthermore, they have developed a technique with which the proportion of said differentiation can freely be changed with RITC medium which contains either no serum or a 0.7 % low-concentration FBS, and with cytokines.

**Table 8 Effects of cytokines on the differentiation of mesenchymal stem cells TBR10-1 toward smooth muscle cells and osteocytes (RITC 80-7, 37°C, 14 days)**

| Differentiation potency | Examp. 13 | Examp. 14 | Examp.15 | Ex.16 | Ex.17 | Compara. Ex.3 |
|---|---|---|---|---|---|---|
| | OSM (10 ng/ml) * | BMP-2 (20 ng/ml) | BMP-4 (20 ng/ml) | GDF-5 (100 ng/ml) | TGF-β2 (10 ng/ml) | |
| Smooth muscles (sm-MLCK) | ― | + + | + + | + + | ― | ± |
| Ossification (% of cells with ALP activity) | 10 | 0 | 2 | 9 | 0 | 0 |

| | | | | | | |
|---|---|---|---|---|---|---|
| *: Parenthetical values show ultimate concentration in medium to which each cytokine was added. | | | | | | |
| Examp. and Ex. denote Example, and Compara. Ex. denotes Comparative Example. | | | | | | |
| Marks: The degree of expression of sm-MLCK as a marker of differentiation toward smooth muscles as shown in Fig. 5(a), detected by chemiluminescence. + +: slightly strong expression; ±: feeble but obvious expression: -: no expression at all | | | | | | |

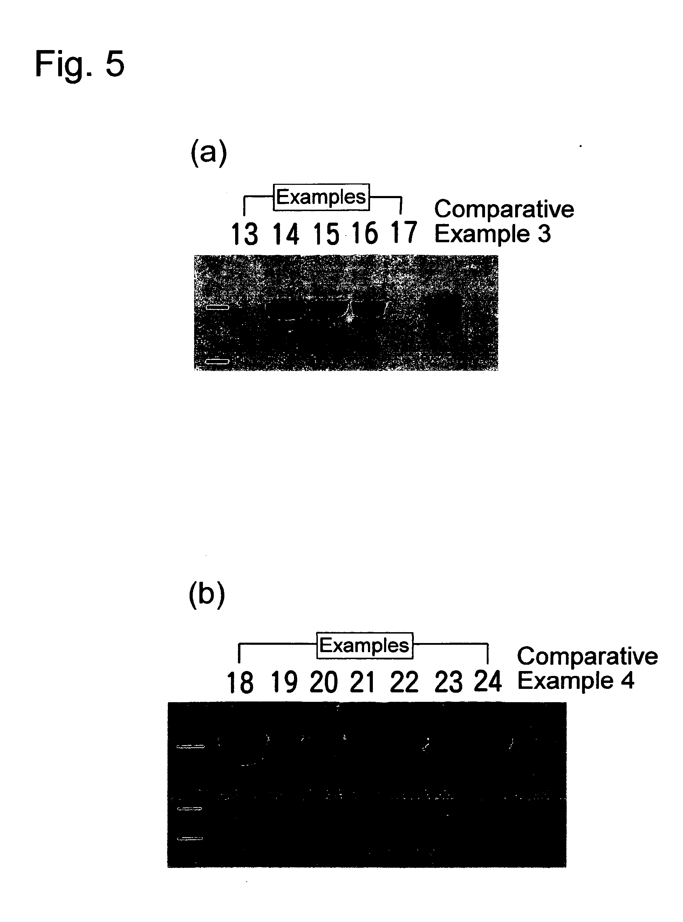

In detail, as shown by the results of expression of myosin L chain kinase as a marker of differentiation toward smooth muscle cells and by the results of differentiation toward ossified cells (Examples 13 to 17; Table 8 and Figure 5a), the differentiation of said cell line toward smooth muscles is inhibited by OSM and TGF-β2. In the presence of BMP-2, BMP-4 and GDF-5, however, differentiation toward smooth muscles is promoted. Differentiation toward ossified cells, on the other hand, is promoted in the presence of OSM and GDF-5. Thus, differentiation toward smooth muscles and differentiation toward ossified cells are oppositely induced by OSM. In other words, differentiation toward smooth muscles and differentiation toward ossified cells are controlled in a closely related manner. There is a possibility that stem cells have different stages of sensitivity depending on different differentiation, i.e., differentiation toward smooth muscles and toward ossified cells.

In the case of TBR10-1, the development process of said stem cells can be roughly classified into four stages as shown in Figure 3. As for details of each growth phase, see the explanation about growth phase of TBR32-1.

As shown in Examples 18-24 (see Table 9 below and Figure 5b), when fed with cytokines at various stages in development process, cell strain of TBR10-1 is differentiated quite differently from the case where cytokines are given continuously. In detail, when BMP-2 is given continuously, differentiation toward smooth muscles proceeds predominantly. BMP-2, however, does not necessarily need to be continuously given for the effect of promotion of differentiation toward smooth muscles to be produced. When given only in 3^{rd} development stage wherein cells grow increasingly and/or in 4^{th} development stage wherein cells are matured, BMP-2 exhibits predominant effect of promotion of differentiation toward smooth muscles, whereas in 1^{st} and 2^{nd} development stage wherein cells are unmatured, BMP-2 has no effect of promotion of differentiation toward smooth muscles (Example 19).

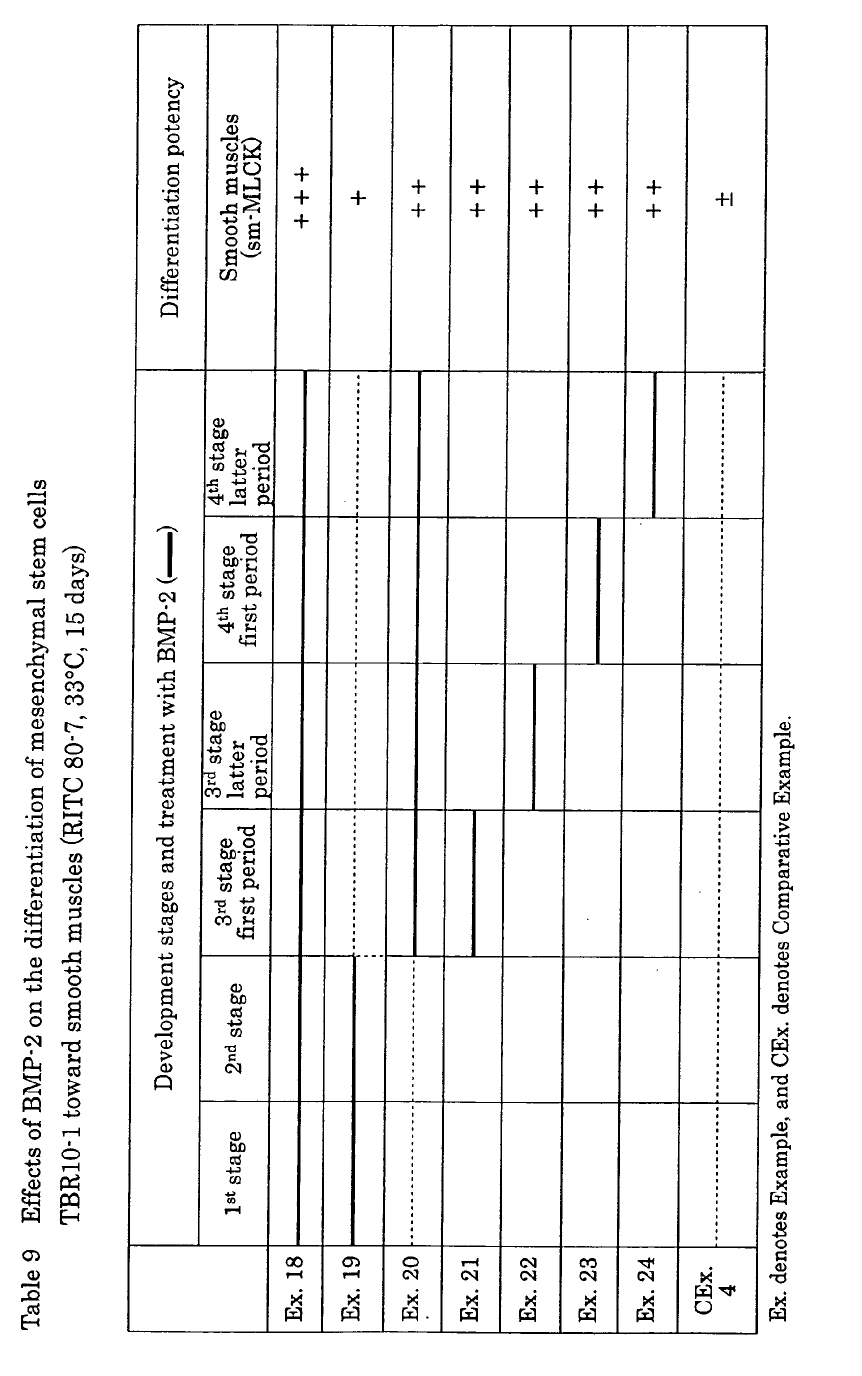

- Marks:: The degree of expression of α-sm-actin as a marker of differentiation toward smooth muscles as shown in Fig. 5(b), detected by chemiluminescence. + + +: strong expression; + +: slightly strong expression; +: obvious expression; ±: feeble but obvious expression: ―: no expression at all

Thus, as is seen in Examples (6 to 12) of effects of OSM on differentiation toward adipocytes, when new drugs such as differentiation-inducing substance or differentiation-inhibiting substance are to be developed from a culture system of stem cells with use of microwell or the like, it is important which development stage of the stem cells to choose for the evaluation of the new drugs; the evaluation of specimen to be screened may be opposite according to the development stage chosen. When, on the other hand, cells are to be provided for the purpose of cell-transplantation in the field of regenerative medicine, cells at optimal differentiation stage will be provided if the choice of species of cytokines, the concentration of cytokines and the timing of addition of cytokines are controlled in cell cultivation. For example, it would be possible to provide cells which have a moderate degree of potency for differentiation toward endothelial cells and smooth muscles which form blood vessel, or cells which have a moderate degree of potency for differentiation toward endothelial cells, smooth muscles and cardiac cells.

### Example 25: Confirmation that cell line TBR52 has pluripotency

(1) In this Example, cell strain TBR52 (see the above-mentioned Non-Patent Documents 3 and 4) was used, and, for a medium, α-MEM medium of formulation as shown in Table 10 below was employed.

**Table 1 α-MEM medium**

| Item | Amount added per 1000 ml of Milli Q Ultrapure Water | Notes |
|---|---|---|
| Sodium bicarbonate | 2.2 g | Manufactured by Wako Pure Chemical Industries, Ltd. (Extra Pure Grade) |
| α-MEM | 10.1 g | Manufactured by Wako Pure Chemical Industries, Ltd. (Extra Pure Grade) |
| 2-Mercaptoethanol (100 mM) | 0.25 ml | Sigma No. M-6250 |
| FBS (fetal bovine serum) | 1.00 ml | Gibco. BRL. Lot No. A0247611 |

### (2) Differentiation potency of TBR52

Cells were identified by a combination of methods such as western blotting (WB), immunostaining and reverse transcription polymerase chain reaction (RT-PCR) as follows.
(2-1) With regard to smooth muscle cells, mononuclear and spindle-shaped cells were microscopically identified in a population of cells which had been cultivated in a 35 mm-plastic dish. For the purpose of detecting, from among a protein mixture which had been extracted from the population of cultivated cells, myosin L chain kinase (sm-MLCK) which specifically expresses in smooth muscle cells, protein was separated by SDS-polyacrylamide gel electrophoresis, and thus separated protein in gel was transblotted onto membrane (WB method), and, thus, smooth muscle cells were identified with use of primary antibody (monoclonal anti-myosin light chain kinase manufactured by Sigma, mouse IgG 2b, clone K36, Product No. M7905) which specifically reacted with sm-MLCK on the membrane, and with use of horseradish peroxidase (HRP)-labeled secondary antibody (goat, anti-mouse IgG F(ab')2, manufactured by ICN, IgG Catalogue No. 55553) by which to detect the reacted primary antibody.
(2-2) With regard to skeletal muscle cells, multinuclear and myotube-shaped cells having a striated muscle structure were microscopically identified in a population of cells which had been cultivated in a 35 mm-plastic dish. For the purpose of detecting, from among a protein mixture which had been extracted from the population of cultivated cells, skeletal myosin Heavy chain II, fast, which specifically expresses in skeletal muscle cells, protein was separated by SDS-polyacrylamide gel electrophoresis, and, thus, skeletal muscle cells were identified with use of primary antibody (monoclonal anti-skeletal myosin fast manufactured by Sigma, mouse IgG, clone MY32, Product No. M4276) which specifically reacted with said myosin Heavy chain II, and with use of horseradish peroxidase (HRP)-labeled secondary antibody (goat, anti-mouse IgG F(ab')2, manufactured by ICN, IgG Catalogue No. 55553) by which to detect the reacted primary antibody.
   For the purpose of observation of cell nuclei in cultivating dish, propidium iodide (PI) as a staining solution whose concentration had been regulated to 1 g/ml was added after culture medium had been removed, and, after a reaction treatment with the staining solution for five minutes at a room temperature, the staining solution was removed, and, then, cells were washed with phosphate buffer solution (PBS). Washing treatment with PBS was conducted three times, each for five minutes.
   The expression of protein of skeletal myosin Heavy chain II, fast, was confirmed by immunostaining according to conventional method. Concretely, a population of cells cultivated on a 25-mm poly-L lysine-coated cover glass (4925-040 manufactured by Asahi TechnoGlass Corporation, Tokyo) in a 35 mm-plastic dish was washed with PBS, and was then fixed at 4°C for 15 minutes with 1.5 ml of 2.5 % paraformaldehyde. After washed with PBS three times, each for five minutes, the cells were treated at room temperature for three minutes with 1.5 ml PBS which contained 0.1 % Triton X-100. After washing with PBS three times, each for five minutes, 1.5 ml of PBS which contained 5 % skim milk (hereinafter referred to as 5 % skim milk-PBS) was added, with which the cells were treated at 4°C for 30 minutes. Monoclonal anti-skeletal myosin fast (manufactured by Sigma, mouse IgG, clone MY32, Product No. M4276) which specifically reacted with said myosin Heavy chain II was diluted to 1/50 concentration with 5 % skim milk-PBS, for use as a primary antibody. The above-mentioned 1.5 ml of 5 % skim milk-PBS which was used for the 30-minute treatment was replaced with 1.5 ml of this primary antibody, with which the cells were allowed to react at 4°C for about 14 hours. Subsequently, the cells were washed with PBS three times, each for five minutes. For secondary antibody, fluorescein isothiocyanate (FITC)-labeled anti-mouse antibody (goat, anti-mouse IgG Fab, manufactured by Sigma, F8711) was diluted to 1/200 concentration with 5 % skim milk-PBS. The cells were allowed to react with 1.5 ml of this secondary antibody at 4°C for 30 minutes, and were then washed with PBS three times, each for five minutes. Subsequently, the cover glass with cells thereon were reversed and mounted on the slide glass with Vectashield (H-1000 manufactured by Vector Laboratories, Burlingame, USA) in between. Thus, the expression of protein of skeletal myosin Heavy chain II, fast, was confirmed with an inverted fluorescence microscope.
(2-3) With regard to cardiomyocites, mononuclear and rod-shaped cells which showed autonomic and periodic contraction (beat) were microscopically identified in a population of cells which had been cultivated in a 35 mm-plastic dish. Thus, the expression of connexin 43 protein which specifically appears in cardiac muscle was confirmed with WB in the same process as mentioned above, except that anti-connexin 43 (manufactured by Chemicon, Product No. MAB3068) was used as primary antibody, and that anti-mouse secondary antibody (goat, anti-mouse IgG F(ab')2, manufactured by ICN, IgG Catalogue No. 55553) was used. Furthermore, the expression of Nkx 2.5 gene and of α myosin Heavy chain gene was confirmed by RT-PCT method, and, thus, cardiomyocites were identified.
   The above-mentioned confirmation was conducted in accordance with manual attached to RT-PCT kit (manufactured by GibcoBRL, Product No. 11904-018). In detail, culture medium was removed from the cultivating dish, and, then, cells adhered to dish bottom were washed with PBS three times. Subsequently, total RNA in thus washed cells were recovered by guanidine thiocyanate·phenol·chloroform method with use of RNA extraction reagent Isogen (manufactured by Nippon Gene Co., Ltd.). From 2.5 µg of thus recovered total RNA, cDNA was prepared by Oligo (dT) method in accordance with manual attached to RT-PCT kit, and was then regulated to 1/10 concentration with DEPC water. With use of 1 µl of this cDNA as a template, there was prepared a reaction liquid of total amount of 10 µl which comprised 1 µl of 10 x PCR buffer, 1 µl of 25 mM MgCl₂, 0.5 µl of 8 mM dNTP, 0.5 µl (10 pmol/µl) of forward primer, 0.5 µl (10 pmol/µl) of reverse primer, 0.1 µl of AmpliTaq Gold DNA Polymerase (manufactured by Applied Biosystems Japan Ltd., Tokyo) and 5.4 µl of DEPC water, and, thus, gene amplification reaction (PCR) was conducted. For PCR, GenAmp 9700 thermal cycler (manufactured by Applied Biosystems Japan Ltd., Tokyo) was used. After initial condition of 95°C for five minutes, 30 cycles of reactions were conducted under predetermined conditions. Subsequently, the above-mentioned liquid was subjected to electrophoresis in 2 % agarose gel with standard marker, and, thus, PCR product was confirmed under UV light. Primers used for the gene amplification reaction had base sequences as follows: 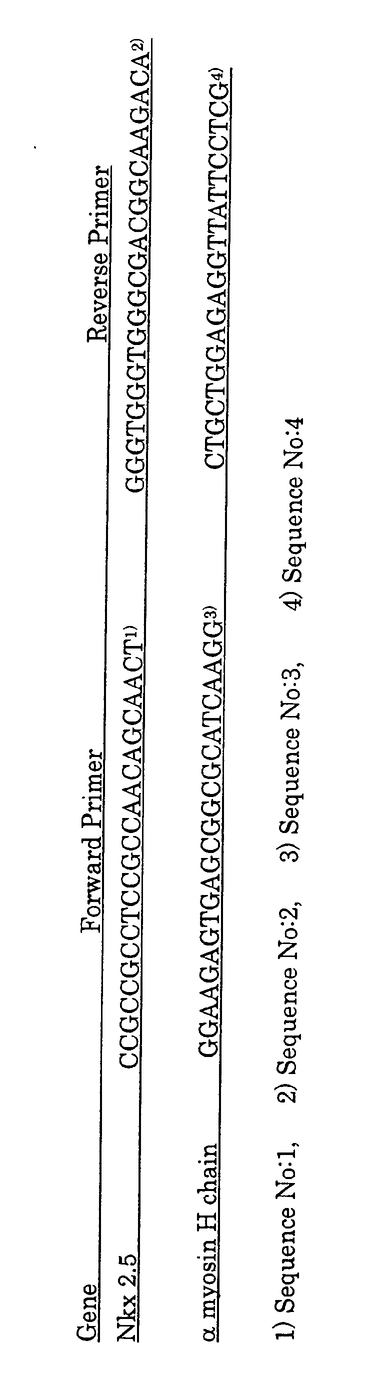
(2-4) With regard to endothelial cells, cobblestone-shaped cells were microscopically identified in a population of cells which had been cultivated in a 35 mm-plastic dish. Endothelial cells were identified by the confirmation of their ability of the uptake of acetylated low-density lipoprotein labeled with 1,1'-dioctadecyl-3,3,3'-tetramethyl-indocarbo-cyanine perchlorate (DiI-Ac-LDL: Product No. L-3484, manufactured by Molecular Probes, USA). In more detail, culture medium in a cultivating dish was sucked up and disposed of, so that cells might not be injured. Then, DiI-Ac-LDL which had been adjusted to 10 µg/ml was added to a serum-free medium, and, thus, the cells were cultivated therein at 33°C for four hours. DiI-Ac-LDL which had not been taken up by the cells was removed by washing with PBS three times, each for five minutes. Thereafter, a 3 % formaldehyde solution was added, and, subsequently, the cells were left to stand still at room temperature for 10 minutes, and were thereby fixed. Then, after washed with distilled water, the cells were observed by a fluorescence microscope with rhodamine filter.
(2-5) With regard to adipocytes, cells having lipid droplet were microscopically observed in a population of cultivated cells, and were identified as adipocytes by an oilred staining method. Culture medium in a cultivating dish was disposed of, and cells which had adhered to dish bottom were washed with PBS eight times, each for eight minutes. Thereafter, with a 3 % formalin solution, the cells were left to stand still at room temperature for 10 minutes, and were thereby fixed. Then, after the formalin solution was substituted by 80 % isopropanol, the cells were left to stand still for a period of one minute, and were subsequently stained with oil red O at 37°C for 10 minutes. After the oilred staining liquid was substituted with 60 % isopropanol, the cells were left to stand still for one minute. Then, after washed twice, each for three minutes, the cells were finally treated with Meyer's hematoxylin solution (manufactured by Wako Pure Chemical Industries, Ltd.) for the sake of nucleus staining. The cells were then washed with running water for two minutes, and, then, adipocytes stained in red were microscopically observed. Incidentally, when grown and matured, adipocytes have remarkable lipid droplet. Hence, even without staining, it would have been easy to identify the cells as adipocytes.

### Examples 26 to 30: Control of differentiation by cytokine

Cytokine to be tested was added to RITC 80-7 medium of the formulation as shown by Table 11 below, and, thus, effects of addition of cytokines were confirmed.

**Table 2 RITC 80-7 medium**

| Item | Amount added per 1000 ml of Milli Q Ultrapure Water | Notes |
|---|---|---|
| Sodium hydroxide | 0.3 g | Manufactured by Wako Pure Chemical Industries, Ltd. (Extra Pure Grade) |
| Sodium bicarbonate | 1.4 g | Manufactured by Wako Pure Chemical Industries, Ltd. (Extra Pure Grade) |
| HEPES | 3.3 g | Sigma No. H-3375 |
| RITC 80-7 | 9.83 g | Iwaki Glass Co., Ltd. No. 99-591-PS |
| Transferrin (10 mg/ml) | 1 ml | Itoham Foods Inc. No. 30601293 |
| rh EGF (10 µg/ml) | 1 ml | RSD. No. 236-EG-200 |
| Insulin (1 mg/ml) | 1 ml | Shimizu Seiyaku Co., Ltd. Batch No. DC18B |
| FBS (fetal bovine serum) | 20 ml | Gibco. BRL. Lot No. A0247611 |

With use of RITC 80-7 medium, TBR52 cells were cultivated in a 90 mm-plastic dish up to sub-confluent state (or confluent of about 80 %), i.e., a state immediately before cells came into contact with one another. After culture medium was removed, 1 ml of EDTA·trypsin solution (a solution of 0.02 g of EDTA·3Na/100 ml of PBS and 0.002 g of trypsin/100 ml of PBS) was added, and the cells were thereby washed. This washing treatment was rapidly conducted twice before the cells were peeled off. In detail, trypsin solution was added, and, immediately after the trypsin solution was spread uniformly on the surface of the cells, the trypsin solution was sucked up quickly with a Pasteur pipette, and was thereby removed. Then, after it was microscopically confirmed that cells had been completely peeled off, α-MEM medium as shown in Table 10 was added, and, thus, the concentration of cells was adjusted to 3 x 10⁵/ml. To 35 mm-plastic dish, 1.5 ml of the resultant cell suspension liquid was added. On the 35 mm-plastic dish, a 25-mm poly-L lysine-coated cover glass (4925-040 manufactured by Asahi TechnoGlass Corporation, Tokyo) had previously been laid. Twenty-four hours after added, α-MEM medium was replaced with a new one. Medium was replaced every two or three days, over a period of 30 days.

With regard to the effects of addition of cytokines on the differentiation potency of cells, α-MEM medium was used for cell cultivation. Twenty-four hours after added, α-MEM medium was replaced with a new one. Medium was replaced every two or three days, over a period of 30 days. Each time medium was replaced, cytokines were added in an amount as shown in Table 12 below.

**Table 3 Amount of cytokines added**

| Kinds | Concentration as prepared | Amount added/1.5 ml of medium | Final concentration | Notes |
|---|---|---|---|---|
| BMP-2 | 10 µg/ml | 3 µl | (20 ng/ml) | Made by RSD |
| OSM | 25 µg/ml | 0.6 µl | (10 ng/ml) | Made by RSD |
| GDF-5 | 50 µg/ml | 0.3 µl | (10 ng/ml) | Made by RSD |
| TGF-β2 | 1 µg/ml | 7.5 µl | (5 ng/ml) | Made by RSD |
| BMP-4 | 10 µg/ml | 3 µl | (20 ng/ml) | Made by RSD |

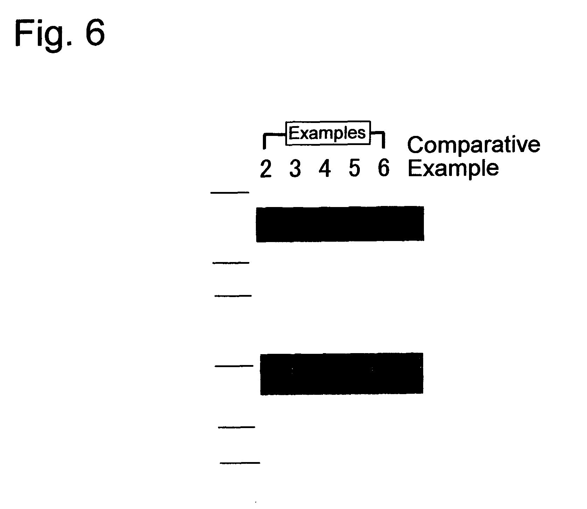

The differentiation of cells was judged by the following criterion. With regard to smooth muscle cells, mononuclear and spindle-shaped cells were microscopically observed. Area occupied by each population of smooth muscle cells per unit area of dish was visually measured, and % average was calculated. Furthermore, from among a protein mixture which had been extracted from the population of cultivated cells, the expression of myosin L chain kinase which specifically expresses in smooth muscle cells was judged by the above-mentioned WB method. With regard to skeletal muscle cells, multinuclear and myotube-shaped cells having a striated muscle structure were microscopically observed. Area occupied by each population of skeletal muscle cells per unit area of dish was visually measured, and % average was calculated.
Furthermore, from among a protein mixture which had been extracted from the population of cultivated cells, skeletal myosin Heavy chain II, fast, which specifically expresses in skeletal muscle cells was judged by the above-mentioned WB method. Photograph in place of drawings to give the results of WB is shown as Figure 6. With regard to cardiomyocites, mononuclear and rod-shaped cells which showed autonomic and periodic contraction (beat) were microscopically observed. The number of long and thin rod-shaped cells which showed autonomic and periodic beat was calculated per unit area, and average was found. With regard to endothelial cells, cobblestone-shaped cells were microscopically observed. Area occupied by each population of endothelial cells per unit area of dish was visually measured, and % average was calculated. With regard to adipocytes, the number of cells which had formed lipid droplets was measured, and average was found. Results are shown in Tables 13 and 14 below.

**Table 4 Differentiation potency of multipotent stem cells**

| Differentiation potency | Ex.2 BMP-2 | Ex.3 OSM | Ex.4 GDF-5 | Ex.5 TGF-β2 | Ex.6 BMP-4 | Comp. Ex. |
|---|---|---|---|---|---|---|
| Smooth muscle cells (%) | 25 | 30 | 24 | 10 | 30 | 20 |
| Skeletal muscle cells | 3.2 | 0 | 4.0 | 0 | 3.0 | 4.4 |
| Cardiac muscles (number of beating cells) | 27.8 | 1.2 | 4.8 | 0 | 1.4 | 2.5 |
| Endothelium (%) | 32 | 76 | 67 | 73 | 74 | 75 |
| Number of adipocytes | 5.6** | 0 | 14.0 | 0 | 101.8 | 8.2 |

| | | | | | | |
|---|---|---|---|---|---|---|
| Unit area: 3.8 mm²; **: Unmatured | | | | | | |
| Ex. denotes Example, and Comp. Ex. denotes Comparative Example. | | | | | | |

**Table 5 Results of WB (transferred from Figure 1)**

| Differentiation potency | Ex.2 BMP-2 | Ex.3 OSM | Ex.4 GDF-5 | Ex.5 TGF-β2 | Ex.6 BMP-4 | Comp. Ex. |
|---|---|---|---|---|---|---|
| Smooth muscle cells | + + | + + | + + | ± | + | + + |
| Skeletal muscle cells | + + + | ― | + | + + | ± | + + |

As is seen in Tables 13 and 14, differentiation toward cardiomyocites in Example 26 where BMP-2 was added to medium was promoted about 10 times as compared with Comparative Example where BMP-2 was not added. Differentiation toward skeletal muscle cells was not clearly seen by microscopic morphological observation since skeletal muscle cells were covered with cardiomyocites. From the results of western blotting analysis, however, differentiation toward skeletal muscle cells was found to have highly been promoted. As for differentiation toward smooth muscle cells and toward endothelial cells, the addition of BMP-2 had no remarkable effects. With regard to differentiation toward adipocytes, there was almost no difference in the number of cells between Example 26 and Comparative Example. There were, however, observed unmatured cells having small lipid droplets, which fact shows that the addition of BMP-2 inhibited differentiation toward adipocytes.

In Example 27 where OSM was added to medium, differentiation toward cardiomyocites was kept below 1/2 of Comparative Example. Differentiation toward skeletal muscle cells and toward adipocytes was almost completely inhibited. Also by microscopic morphological observation, neither multinuclear and myotube-shaped skeletal muscle nor adipocytes having lipid droplets were identified. This effect of inhibition on differentiation toward skeletal muscle cells was confirmed also by the analysis of expression of protein of skeletal myosin Heavy chain II, fast, by means of western blotting. As for differentiation toward smooth muscle cells and toward endothelial cells, however, the addition of OSM had almost no effects.

In Example 28, the addition of GDF-5 to the medium slightly promoted differentiation toward cardiomyocites and toward adipocytes. The addition of GDF-5, however, inhibited the expression of protein of skeletal myosin Heavy chain II, fast, which is specific to skeletal muscle cells. As for differentiation toward smooth muscle cells and toward endothelial cells, the addition of GDF-5 had no effects at all.

In Example 29, the addition of TGF-β2 to the medium inhibited differentiation toward smooth muscle cells, cardiomyocites and adipocytes, whereas it had no effects on differentiation toward endothelial cells. As for differentiation toward skeletal muscle cells, there were identified no cells having a form of skeletal muscle cell from microscopic morphological observation. In western blotting, however, the expression of protein of skeletal myosin Heavy chain II, fast, which is specific to skeletal muscle cells was promotive. The expression of said protein cannot precisely be explained at this point of time. It may, however, be a clue to clarifying the relation between morphological cell fusion which occurs in the course of differentiation toward skeletal muscle cells and the period of initial induction for differentiation toward skeletal muscle cells.

In Example 30 where BMP-4 was added to the medium, differentiation toward adipocytes was promoted more than 10 times as compared with Comparative Example where no BMP-4 was added. Differentiation toward smooth muscle cells and skeletal muscle cells, on the other hand, was found to be inhibited, from the analysis of western blotting. Differentiation toward cardiomyocites was found to be inhibited, since only few cells showed autonomic beat which is a characteristic feature of cardiomyocites. On differentiation toward endothelial cells, the addition of BMP-4 had almost no effects.

The above-mentioned results show that the addition of cytokines to multipotent stem cells makes it possible to predominantly amplify or reduce differentiation in a direction toward certain specific cells. The above results also show that it may be possible to inhibit undesired cells and amplify only desired cells, depending on how to combine cytokines to be used.

## Claims

1. A method to induce the differentiation of multipotent stem cells by bringing said cells into contact with a pharmacological agent during the process of growth of said multipotent stem cells, wherein said cells are brought into contact with a pharmacological agent in at most four of the growth phases which comprise i) 1^{st} development stage, ii) 2^{nd} development stage, iii) the first period of 3^{rd} development stage, iv) the latter period of 3^{rd} development stage, v) the first period of 4^{th} development stage, and vi) the latter period of 4^{th} development stage, and wherein said agent is a substance which is capable of promoting and/or inhibiting the differentiation of said cells in at least two directions.

2. A method of claim 1 to induce the differentiation of cells wherein the multipotent stem cells are bone marrow stromal cells.

3. A method of claim 2 to induce the differentiation of cells wherein the bone marrow stromal cells have been derived from temperature-sensitive SV-40 T-antigen gene transgenic mice.

4. A method of claim 1 or 2 to induce the differentiation of cells wherein cells are differentiated toward at least two of smooth muscle cells, osteoblasts and adipocytes.

5. A method of anyone of claims 1 to 3 to induce the differentiation of cells wherein the pharmacological agent is cytokine which is capable of promoting or inhibiting the differentiation of cells.

6. A method of claim 4 to induce the differentiation of cells wherein the cytokine is selected from the group consisting of oncostatin M (OSM), bone morphogenetic protein-2 (BMP-2), bone morphogenetic protein-4 (BMP-4), growth differentiation factor 5 (GDF-5) and transforming growth factor (TGF-β2).

7. A method for evaluation of the ability of a pharmacological agent to promote or inhibit the differentiation of cells with use of a method to induce the differentiation of multipotent stem cells by bringing said cells into contact with a pharmacological agent during the process of growth of said cells, wherein said cells are brought into contact with the agent in at most four of the growth phases which comprise i) 1^{st} development stage, ii) 2^{nd} development stage, iii) the first period of 3^{rd} development stage, iv) the latter period of 3^{rd} development stage, v) the first period of 4^{th} development stage, and vi) the latter period of 4^{th} development stage, and wherein said agent is a proposed substance which is expected to promote and/or inhibit the differentiation of said cells in at least two directions.

8. A method for evaluation of claim 7 wherein the multipotent stem cells have been derived from temperature-sensitive SV-40 T-antigen gene transgenic mice.

9. A method for evaluation of claim 7 or 8 wherein the degree of differentiation of the multipotent stem cells which is caused by a proposed agent is to be compared with the degree of differentiation which is caused by bringing said cells, during the process of growth of the cells, into contact with cytokine which is capable of promoting or inhibiting the differentiation of the cells.

10. A method for evaluation of claim 9 wherein the cytokine is selected from the group consisting of oncostatin M (OSM), bone morphogenetic protein-2 (BMP-2), bone morphogenetic protein-4 (BMP-4), growth differentiation factor 5 (GDF-5) and transforming growth factor (TGF-β2).

11. A preparation for regenerative medicine which mainly comprises cells which have been induced by a method of anyone of claims 1, 2 and 4 to 6 to induce cells.

12. A preparation of claim 11 wherein the cells are originated from mammals.

13. A set of cytokines to regulate the differentiation of cells of mammals, which comprises a combination of two or more cytokines as an effective ingredient, and which is capable of determining three or more directions of differentiation of multipotent stem cells including bone marrow stromal cells, and is capable of regulating the degree of differentiation in each of cells whose differentiation direction has been determined.

14. A set of cytokines of claim 13 wherein the multipotent stem cells are bone marrow stromal cells, and wherein the cells are differentiated in three or more directions.

15. A set of cytokines of claim 14 or 15 wherein the cells are differentiated toward smooth muscle cells, skeletal muscle cells, cardiomyocytes, endothelial cells or adipocytes.

16. A set of cytokines of anyone of claims 13 to 15 wherein the degree of each differentiation is promoted or inhibited by at least 10 % in comparison with the degree of any differentiation which is caused in the presence of serum.

17. A set of cytokines of anyone of claims 13 to 16 wherein the combined two or more cytokines are selected from the group consisting of bone morphogenetic protein-2 (BMP-2), bone morphogenetic protein-4 (BMP-4), oncostatin M (OSM), growth differentiation factor 5 (GDF-5) and transforming growth factor (TGF-β2).

18. A set of cytokines of claim 17 wherein a combination of cytokines is selected from the group consisting of BMP-2 and BMP-4; BMP-2 and OSM; BMP-2 and TGF-β2; BMP-2, BMP-4 and OSM; OSM-BMP-4; OSM and TGF-β2; OSM and GDF-5; OSM, GDF-5 and BMP-4; OSM, GDF-5, TGF-β2 and BMP-4; BMP-2, OSM, GDF-5 and BMP-4; and BMP-2, OSM, GDF-5, TGF-β2 and BMP-4.

19. A set of cytokines of anyone of claims 13 to 17 wherein the bone marrow stromal cells are multipotent adult stem cells which can be differentiated toward at least smooth muscle cells, beating cardiomyocytes and endothelial cells with the stimulus of BMP-2.

20. A set of cytokines of anyone of claims 1 to 6 wherein the bone marrow stromal cells have been derived from temperature-sensitive SV-40 T-antigen gene transgenic mice.

21. A set of cytokines of anyone of claims 1 to 8 wherein the differentiation of bone marrow stromal cells is induced in an environment which is selected from the group consisting of *in vitro, ex vivo* and *in vivo*.

22. A set of cytokines of anyone of claims 13 to 19 wherein the *ex vivo* or *in vivo* differentiation of bone marrow stromal cells is employed for the transplantation of cells in regenerative medicine.

23. A set of cytokines of claim 20 wherein the *in vitro* differentiation of bone marrow stromal cells is employed for the screening of a pharmacological agent which is capable of differentiating said cells.

24. A method for the screening of medicines which act on the differentiation potency of vertebrate cells, wherein (A) multipotent bone marrow stromal cells derived from temperature-sensitive SV-40 T-antigen gene transgenic mice are prepared, (B) said cells are cultivated in a medium in which the cells may be proliferated in the presence of a proposed agent which is expected to be able to differentiate the cells, (C) the direction or degree of differentiation of thus cultivated cells is determined, and (D) the result of thus determined direction or degree of differentiation is compared with the result of cultivation of said cells in the absence of said agent, and, then, the difference between said two results is used as an index to the action of said agent on the differentiation potency of bone marrow stromal cells.

25. A method of claim 24 wherein at least two which are selected from the group consisting of BMP-2, BMP-4, OSM, GDF-5 and TGF-β2 are used as comparative agents.

26. A method for screening of claim 25 wherein cell cultivation is conducted in a serum-free medium.


SEQUENCE LISTING

<110> JAPAN SCIENCE AND TECHNOLOGY AGENCY

<120> INDUCTION OF DIFFERENTIATION OF STEM CELLS, AND CONTROL
      OF DIFFERENTIATION POTENCY OF STEM CELLS

<130> 15833EP

<140> 04 722 990.1
<141> 2004-03-24

<150> JP2003-83106
<151> 2003-03-25

<150> JP2003-95242
<151> 2003-03-31

<160> 4

<170> PatentIn Ver. 2.1

<210> 1
<211> 24
<212> DNA
<213> Artificial Sequence

<220>
<223> Description of Artificial Sequence:Primer,
      synthesized on the basis of a sequence of 1,108th
      to 1,521st bases of murine Nkx 2.5 gene. (NCBI
      Accession No.NM008700)

<400> 1
ccgccgcctc cgccaacagc aact                                        24


<210> 2
<211> 24
<212> DNA
<213> Artificial Sequence

<220>
<223> Description of Artificial Sequence:Primer,
      synthesized on the basis of a sequence of 1,108th
      to 1,521st bases of murine Nkx 2.5 gene. (NCBI
      Accession No.NM008700)

<400> 2
gggtgggtgg gcgacggcaa gaca                                              24

<210> 3
<211> 24
<212> DNA
<213> Artificial Sequence

<220>
<223> Description of Artificial Sequence:Primer,
      synthesized on the basis of a sequence of 5,630th
      to 5,931st bases of murine gene which encodes
      alpha myosin H chain. (NCBI Accession No.M76601)

<400> 3
ggaagagtga gcggcgcatc aagg                                              24

<210> 4
<211> 22
<212> DNA
<213> Artificial Sequence

<220>
<223> Description of Artificial Sequence:Primer,
      synthesized on the basis of a sequence of 5,630th
      to 5,931st bases of murine gene which encodes
      alpha myosin H chain. (NCBI Accession No.M76601)

<400> 4
ctgctggaga ggttattcct cg                                                22